(19) **Europäisches Patentamt / European Patent Office / Office européen des brevets**

(11) **EP 4 458 798 A1**

(12) # EUROPEAN PATENT APPLICATION

(43) Date of publication:
**06.11.2024 Bulletin 2024/45**

(21) Application number: **24166036.4**

(22) Date of filing: **25.03.2024**

(51) International Patent Classification (IPC):
*C07C 51/00* (2006.01)    *C07C 51/44* (2006.01)
*C07C 51/48* (2006.01)    *C07C 59/185* (2006.01)

(52) Cooperative Patent Classification (CPC):
(C-Sets available)
**C07C 51/00; C07C 51/44; C07C 51/48**    (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **12.04.2023 IN 202321027132**

(71) Applicants:
• **Indian Oil Corporation Limited**
  **400 051 Mumbai Bandra (East) (IN)**
• **Department Of Biotechnology**
  **Ministry Of Science & Technology**
  **New Delhi - 110 003 (IN)**

(72) Inventors:
• **SATLEWAL, Alok**
  **121007 Haryana (IN)**
• **AGRAWAL, Ruchi**
  **121007 Haryana (IN)**
• **CHAUHAN, Prakram Singh**
  **121007 Haryana (IN)**
• **KUMAR, Ravindra**
  **121007 Haryana (IN)**
• **GUPTA, Ravi Prakash**
  **121007 Haryana (IN)**
• **RAMAKUMAR, Sankara Sri Venkata**
  **121007 Haryana (IN)**

(74) Representative: **Turner, Craig Robert**
  **AA Thornton IP LLP**
  **8th Floor, 125 Old Broad Street**
  **London EC2N 1AR (GB)**

(54) **AN INTEGRATED PROCESS FOR THE PURIFICATION OF LEVULINIC ACID FROM TECHNICAL LIGNIN**

(57) The present invention relates to a novel integrated process to extract and purify levulinic acid (LA) produced from the technical lignin waste (2-G residue) generated after second generation (2-G) ethanol production from biomass. In this process, liquid-liquid extraction was carried out using fusel oil as an organic extractant to yield a LA rich organic phase and an acidic aqueous phase (raffinate). In addition, selected amines improved the LA yields by acting synergistically. Further fractional distillation was carried out to purify LA. The acidic raffinate is recycled back to the reaction in multiple times, which reduce the use of additional catalyst and make the overall process cost effective. The process is very simple and suitable to remove any colour and humins (soluble tar), which would otherwise form a problem in subsequent process step, particularly in distillation. At optimized process conditions 95% LA is recovered with 98% purity.

EP 4 458 798 A1

(52)  Cooperative Patent Classification (CPC): (Cont.)

C-Sets
**C07C 51/00, C07C 59/185;**
**C07C 51/44, C07C 59/185;**
**C07C 51/48, C07C 59/185**

**Description**

FIELD OF THE INVENTION

The present invention relates to a novel integrated process for the extraction and purification of levulinic acid (LA). More specifically, the present invention relates to a novel and integrated liquid-liquid extraction and purification method to produce highly pure LA from the technical lignin waste (2-G residue) generated after second generation (2-G) ethanol production from lignocellulosic biomass.

BACKGROUND OF THE INVENTION

Levulinic acid (LA) is widely described as one of the high potential platform chemicals which can be derived from lignocellulosic materials/2-G bioethanol residue. It is a carboxylic acid, used in the manufacture of many daily need items. It can be used as a raw material in a variety of industries including plastics, polymers, pharmaceuticals, cosmetics, food, agriculture, biofuel, chemical solvents, rubber manufacturing, fertilizer etc. It can also be used as a platform chemical for the manufacturing of value-added products such as methyltetrahydrofuran (MTHF), a fuel additive; δ-aminolevulinic acid (DALA), a broad-spectrum herbicide/pesticide; or diphenolic acid (DPA); a substitute for bisphenol A in the polymer industry. It is also known as keto acid ($C_5H_8O_3$), is a ketonic group compound which has largely replaced petroleum-based goods in the chemical and biofuel industries. It is a colourless small chain fatty acid, soluble in acidic ethanol, diethyl ether, and water. LA is a common substrate in chemical reactions such as condensation, esterification, halogenation, hydrogenation, oxidative dehydrogenation, and salt formation. It is a very versatile and potential building block for the synthesis of various organic products. Various fuel and chemical products can be obtained from LA. It is considered as the top twelve organic chemicals by the energy department of the USA. Its permeation enhancers are very safe and effective. LA can also be obtained from cellulosic biomass, grass, and wood chips, and has also been regarded as a basic premium material due to its high chemical reactivity. The increasing demand for LA in the plasticizer, pharmaceutical, and cosmetic sectors is the primary factor for its larger market.

To remove and recover carboxylic acids from water, a variety of separation techniques have been used including adsorption, extraction, ion exchange, ultrafiltration, nanofiltration, precipitation, electrodialysis, distillation, membrane separation, and reverse osmosis. Among these techniques, extraction, nanofiltration, electrodialysis, and adsorption have been employed in the separation of LA from aqueous solutions. Few of the process are described as below:

WO2014/037560A1 disclose a process for the separation of LA from a biomass hydrolysate. The process involves a combination of solvent extraction and nanofiltration, whereby the organic phase obtained after solvent extraction is subjected to the nanofiltration. The process allows the removal of humins (soluble tar) and colour, which prevent problems in the subsequent isolation of LA, particularly in distillation. This increases the yield of LA. The process makes use of ability of the solvent in the organic phase to keep the humins in solution.

WO2014087015A1 describes a process for the isolation of LA and formic acid from a composition comprising both. This process comprises a solid-liquid separation step, a vapor removal step, and a solvent-solvent extraction step, wherein a vapor condensate and an aqueous phase from the solvent extraction is used to wash the solid fraction. Washing with a vapor condensate results in higher LA yields compared to washing with normal water. Washing first with aqueous phase and subsequently with vapor condensate results in even higher LA yields. The process is suitable for isolating LA and formic acid from compositions made by acid hydrolysis of a lignocellulosic biomass.

WO2017064069A1 describes a process for the purification of LA comprising the following steps: a. providing a composition 1, comprising at least 75 wt% of LA; b. cooling composition 1 to temperature Tc, 104.5 °C; c. performing melt crystallization of composition 1 comprising the steps of: i. bringing the cooled composition 1 into contact with LA crystal seeds, ii. allowing the LA in composition 1 to crystallize at one temperature Tc to obtain crystals 1 and liquid 1, and iii. draining of liquid 1; d. optionally, treating crystals 1, after draining of liquid 1, by sweating, according to the following steps: i. heating the crystals 1 at a temperature between 5 and 40 °C to obtain crystals 2 and liquid 2, and ii. draining of liquid 2; e. melting the crystals 1 or 2, after draining of liquid 1 or 2, to obtain composition 2, f. determining the LA concentration in composition 2 and, in case the LA concentration is below a predetermined value, repeating steps b, c, optionally d, and e, as many times as necessary to obtain a final composition with a predetermined LA.

US2684982A relates to the recovery and purification of LA from aqueous mixtures by treatment of carbohydrate materials with dilute aqueous solutions of mineral acids which eliminates the necessity of removing insoluble humus materials prior to extraction.

US2257389 relates to separation of LA from the crude solution which contains by-products such as humus by filtration. For this approximately equal volume of methylene chloride has been added in each extraction until the LA has been removed as completely from the aqueous solution. If desired extraction may be carried out in a continuous manner, the methylene chloride preferably being caused to flow in contact with the aqueous solution of LA in a counter-current manner. After extraction has been completed, the LA may be recovered from the methylene chloride extract in relatively pure form by distilling off the methylene chloride to leave the residue of recovered acid. This residue will be found to be substantially free from coloured by-products and may be used as so recovered for any purposes.

The prior art is relevant to purify LA from the aqueous solution using organic solvents, ethers, ionic liquids, or their combinations in multiple steps. Further the techniques and process used in the prior art are expensive, cumbersome, and produces waste material during the process. Further, none of the prior discloses a method for the extraction and purification of LA using fusel oil, which is generated after ethanol distillation as a waste and available cheaply. Therefore, there is a need for a novel, simple, straight forward method applicable to isolate high purity LA, using waste solvent and amine extractant. Further, the process must be eco-friendly and cost-effective in which the used solvent and raffinate (acidic rich aqueous phase) could be recovered easily and recycled multiple times after use without any substantial loss in activity.

## SUMMARY OF THE INVENTION

This summary is provided to introduce a selection of concepts, in a simplified format, that are further described in the detailed description of the invention. This summary is neither intended to identify key or essential inventive concepts of the invention and nor is it intended to determine the scope of the invention.

A novel, integrated, cost-effective, and environmentally benign approach is disclosed in the present invention for the purification of LA using fusel oil, a waste stream generated during distillation process of 2G bioethanol production in the presence of selected amines followed by fractional distillation.

The present invention relates to a liquid-liquid extraction process that has been developed for the recovery (extraction yield 95%) of highly pure (98%) LA wherein, fusel oil and N-methyldioctylamine was employed. Furthermore, raffinate (acidic rich aqueous phase) generated from the down streaming is recycled back to the reaction step to make the overall process economically feasible and eco-friendly.

The present invention also provides a technique for the sustainable biorefinery where apart from 2-G bioethanol, several other valuable products would be obtained by utilizing its own by-products (technical spirit, LA, etc.).

Accordingly, the present invention provides, a novel integrated process for the extraction and purification of levulinic acid (LA) from a technical lignin, wherein the said process comprises of:

    i. heating the technical lignin with a catalyst in water, into a reactor to obtain a supernatant containing LA and a black solid insoluble humins fraction;

    ii. separating the supernatant containing LA and the black solid insoluble humins fraction through centrifugation;

    iii. mixing of the supernatant containing LA with an organic solvent and an amine extractant;

    iv. purifying LA by a reactive extraction using the amine extractant with the organic solvent and separating an acidic aqueous liquid waste or raffinate to obtain a LA rich organic phase,
    wherein the reactive extraction involves a reversible reaction between the LA taken in the aqueous phase and the amine extractant in the organic phase which results in the formation of amine extractant-acid complex such as octylamine-LA complex or N-methyldioctylamine -LA complex, having high affinity for the organic phase;

    v. recycling the acidic aqueous liquid waste or raffinate generated after organic solvent extraction for the next batch of producing LA;

    vi. fractionally distilling the LA rich organic phase to separate the organic solvent and LA; and

    vii. recycling the organic solvent for the next round of extraction and purification.

In another aspect the present invention provides, the technical lignin is bioethanol residue generated from a lignocellulosic biomass after 2-G bioethanol processing.

In another aspect the present invention provides, the lignocellulosic biomass is selected from agricultural residues, grass, fiber process residue, sugar cane straw, sorghum, forestry waste, hardwood, softwood, sawdust, crops or lignocellulosic material and a combination thereof; wherein the agricultural residue is selected form corn stover, wheat straw, cotton stalk, rice straw, canola straw, soybean stover and a combination thereof;

the grass is selected form switchgrass, miscanthus, jatropha cuttings and a combination thereof; and

the fiber process residue is selected form corn fiber, beet pulp, pulp mill fines and rejects, sugar cane bagasse and a combination thereof.

In another aspect the present invention provides, the heating of the technical lignin in step (i) is performed at 50 - 300 °C for 60 - 180 minutes.

In another aspect the present invention provides, the catalyst comprises a combination of 0.5 % - 4 % of concentrated $H_2SO_4$ and 0.5 % - 4 % of $FeCl_3$.

In another aspect the present invention provides, the mixing of the supernatant containing LA with the organic solvent in step (iii) is done at a temperature of 25- 30 °C for 30 - 240 minutes.

In another aspect the present invention provides, the organic solvent is selected from fusel oil, dichloromethane, diethyl ether, 1-butanol, 1-pentanol, ethyl acetate, hexane, chloroform, benzene, toluene and a synthetic blend of fusel oil.

In another aspect the present invention provides, components of fusel oil are iso amyl alcohol (IAA), iso butyl alcohol (IBA), active amyl alcohol (AAA), butyl alcohol (BA), propyl alcohol (PA), and hexanol (H) and mixture of methanol, ethanol, acid, water, and metal salt.

In another aspect the present invention provides, the synthetic blend of fusel oil comprises of iso amyl alcohol (55 -60%), iso butyl alcohol (18 - 20%), active amyl alcohol (10 - 15%), butyl alcohol (5 - 8%), propyl alcohol (5 - 8%), and hexanol (2 - 5%).

In another aspect the present invention provides, the amine extractant is selected from octylamine and N-methyldioctylamine.

In another aspect the present invention provides, the concentration of the amine extractant is in the range of 0.2 - 1.0 M.

In another aspect the present invention provides, the technical lignin is obtained from pre-treatment of lignocellulosic biomass, comprising of:

i. treating lignocellulosic biomass with dilute acid selected from $H_3PO_4$, $HNO_3$, $CH_3COOH$, and $H_2SO_4$ having 0.02 - 3% w/w of the biomass for 5 - 60 min, at temperature of 50 °C-100 °C, followed by heating at 150 °C - 200 °C to obtain a slurry, to hydrolyse 40 % - 90 % of hemicellulose into sugars and to obtain minimum amounts of inhibitors selected from furfurals, hydroxy methyl furfural, levulinic acid, and acetic acids;

ii. cooling the slurry to the temperature of 35 °C - 50 °C to carry out the enzymatic hydrolysis and fermentation either as separate hydrolysis and fermentation (SHF) or simultaneous saccharification and fermentation (SSCF) at a temperature in range of 35 °C - 50 °C to obtain a fermented broth; and

iii. distilling the fermented broth by standard distillation and molecular sieve methods to produce ethanol and technical lignin.

In yet another aspect the present invention provides, the enzymatic hydrolysis and fermentation of lignocellulosic biomass is done with genetically modified yeast *saccharomyces cerevisiae.*

## OBJECTIVES OF THE PRESENT INVENTION

The main objective of the present invention is to provide a novel, environmentally friendly, and economically feasible liquid-liquid extraction process for the extraction and purification of highly pure levulinic acid.

Another objective of the present invention is to extract the levulinic acid from technical lignin or 2-G biorefinery residue produced from lignocellulosic biomass generated in 2-G bioethanol processing.

## ABBREVIATIONS

LA: levulinic acid
FA: formic acid

IAA: iso amyl alcohol
IBA: iso butyl alcohol
AAA: active amyl alcohol
BA: butyl alcohol
PA: propyl alcohol
H: hexanol
SHF: separate hydrolysis and fermentation
SSCF: simultaneous saccharification and fermentation

## DETAILED DESCRIPTION OF THE INVENTION

For the purpose of promoting an understanding of the principles of the invention, reference will now be made to the embodiments in the specific language will be used to describe the same. It will nevertheless be understood that no limitation of the scope of the invention is thereby intended, such alterations and further modifications in the illustrated process, and such further applications of the principles of the invention as illustrated therein being contemplated as would normally occur to one skilled in the art to which the invention relates. Unless otherwise defined, all technical and scientific terms used herein have the same meaning as commonly understood by one of ordinary skilled in the art to which this invention belongs. The composition, methods, and examples provided herein are illustrative only and not intended to be limiting.

The articles "a", "an" and "the" are used to refer to one or to more than one (i.e., to at least one) of the grammatical object of the article.

The terms "comprise" and "comprising" are used in the inclusive, open sense, meaning that additional elements may be included. It is not intended to be construed as "consists of only". Throughout this specification, unless the context requires otherwise the word "comprise", and variations such as "comprises" and "comprising", will be understood to imply the inclusion of a stated element or step or group of element or steps but not the exclusion of any other element or step or group of element or steps.

Unless defined otherwise, all technical and scientific terms used herein have the same meaning as commonly understood by one of ordinary skill in the art to which this disclosure belongs. Although any methods and materials similar or equivalent to those described herein can be used in the practice or testing of the disclosure, the preferred methods and materials are now described. All publications mentioned herein are incorporated herein by reference.

The terminology and structure employed herein is for describing, teaching, and illuminating some embodiments and their specific features and elements and does not limit, restrict, or reduce the spirit and scope of the invention.

The present invention relates to a novel integrated process for the extraction and purification of LA from technical lignin or 2-G biorefinery waste residue which utilizes lignocellulosic biomass as its first step feed.

## 2-G Biorefinery waste residue/technical lignin:

Technical lignin or 2-G biorefinery waste residue accumulates in huge amounts after 2-G bioethanol processing of the lignocellulosic biomass. According to one estimate, about 550 - 600 Kg of technical lignin is generated from each ton of lignocellulosic biomass. Considering this, a commercial scale bioethanol plant capable of processing 500 tons per day (TPD) will generate about 250 TPD technical lignin along with 100 kilo liter per day (KLPD) of ethanol production. This technical lignin does not have any other usage except burning in a boiler to generate heat and power. However, this is generally considered as a waste management practice instead of a valuable or economically profitable proposition for the 2G ethanol production technology. The complete process of generating the technical lignin during the 2G ethanol process is described here.

In one embodiment the present invention provides, the lignocellulosic biomass includes agricultural residues such as corn stover, wheat straw, cotton stalk, rice straw, canola straw, and soybean stover; grass such as switchgrass, miscanthus, jatropha cuttings, fiber process residues such as corn fiber, beet pulp, pulp mill fines and rejects and sugar cane bagasse; sugar cane straw and sorghum; forestry wastes, other hardwoods, softwood and sawdust as well as other crops or sufficiently abundant lignocellulosic material.

In another embodiment the present invention provides, the biomass that is useful for the invention has a relatively high carbohydrate content, is relatively dense, and/or is relatively easy to collect, transport, store and/or handle.

In another embodiment the present invention provides, the biomass that is useful includes corn cobs, corn stover, sugar cane bagasse, sugar cane straw, rice straw, cotton stalk, Jatropha cuttings and switchgrass. The lignocellulosic biomass may be derived from a single source or can comprise a mixture derived from more than one source; for example, biomass could comprise a mixture of corn cobs and corn stover, or a mixture of stems or stalks and leaves. The biomass may be used directly as obtained from the source, or may be subjected to some pre-processing, for example, reduction in size, washing/soaking in hot or cold water to remove dirt, silica or extractives. Drying prior to the pre-treatment may occur as well by conventional means, such as exposure to ambient temperature, to vacuum or flowing air at atmospheric pressure.

**Dilute acid pre-treatment conditions:**

In one embodiment the present invention provides, the lignocellulosic biomass is treated with dilute acid selected from $H_3PO_4$, $HNO_3$, $CH_3COOH$, and $H_2SO_4$. The concentration of the acid added to the lignocellulosic feedstock is between 0.02 - 3% w/w of biomass and the acid treatment is carried out for 5 min to 60 min to obtain a slurry. The acid soaking may be carried out at 50 - 100 °C and followed by heating between 150 - 200 °C.

The pre-treatment temperature utilized in the process will depend on the retention time, acid concentration and the feedstock used. The pre-treatment conditions are applied so as to hydrolyse 40% - 90% of hemicellulose (xylan, arabinan etc.) to sugars and to obtain minimum amounts of inhibitors like furfurals, hydroxy methyl furfural, levulinic acid, and acetic acid, etc.

**Enzymatic saccharification and fermentation:**

In one embodiment the present invention provides, after pre-treatment, slurry is cooled to the temperature of 35 - 50 °C to carry out the enzymatic hydrolysis and fermentation, either as separate hydrolysis and fermentation (SHF) or simultaneous saccharification and fermentation (SSCF). The genetically modified yeast *saccharomyces cerevisiae* is preferentially utilized for hexose and pentose sugars fermentation.

**Distillation and ethanol recovery:**

In one embodiment the present invention provides, the distillation of ethanol from the fermented broth was carried out by standard distillation and molecular sieve methods to produce highly pure ethanol (>99.5). The residue generated after ethanol distillation and recovery was termed as 2-G biorefinery waste residue or technical lignin.

**LA production:**

In one embodiment the present invention provides, technical lignin after enzymatic hydrolysis, fermentation and distillation was stored in a cool and dried place directly after airdrying, for the study. The chemical composition of technical lignin was determined, screening of catalyst, catalyst concentration optimization, synergistic catalysis was discovered, LA production process was optimized at 2 Kg scale.

**Downstreaming and purification of LA:**

In one embodiment the present invention provides, the produced acid is purified by liquid-liquid extraction to yield an organic phase rich with LA, and an aqueous phase (raffinate) in presence of amine extractant and solvent/diluent. Two different amines in combinations with ten different types of diluents (alcohols, esters, alkanes) were experimentally evaluated for reactive extraction of LA. In comparison to only diluents, reactive extractants based on amine-diluent combinations demonstrated higher extractive yields. Extractants with the highest extractive capacity were selected for further research aimed to optimize the concentration of amines for maximum extraction yield. Thereafter, each component (acid/fusel oil) from the organic phase is separated by fractional distillation and selected solvent (fusel oil) was recycled. The process is very suitable to remove any colour and humins (soluble tar), which would otherwise create a problem in subsequent process step, particularly in distillation. The generated acid rich aqueous phase (raffinate) act as a catalyst therefore it was recycled many times. Overall, the process is simple, cost effective, and economically feasible and resulted into higher yields of LA.

**EXAMPLES:**

The present disclosure with reference to the accompanying examples describes the present invention. However, those of skill in the art should, in light of the present disclosure, appreciate that many changes can be made in the specific embodiments which are disclosed and still obtain a like or similar result without departing from the spirit and scope of the invention. It is understood that the examples are provided for the purpose of illustrating the invention only and are not intended to limit the scope of the invention in any way.

**Example 1: LA production process using catalyst from technical lignin**

The LA production process is conducted in a 2L stainless steel batch reactor with a nominal pressure of 10 MPa and temperature of 200 °C. A thermocouple probe inside the reactor is connected to a digital temperature indicator, with an accuracy of $\pm 0.5$ °C. At the beginning of the experiment, the technical lignin and $FeCl_3 + H_2SO_4$ is added into the reactor (Table 1) and tightly sealed. When the temperature inside the reactor reached the desired set-point (200 °C) after several minutes, the time count starts from 0 and the temperature is kept constant. After reaching the target reaction time, the reactor is discharged into a cooling bath to stop the reaction. The supernatant liquid phase is separated through centrifugation which contained LA whereas black solid insoluble fraction is humins. LA is measured for each reaction by HPLC (Waters Gesellschaft Gmbg, Austria) following proper dilution of samples using Bio-Rad Aminex HPX-87H column (Bio-Rad, USA) coupled with refractive index (RI) and Photo Diode Array (PDA) detector at a flow rate of 0.6 ml/min at column temperature of 50 °C. The mobile phase used is 0.005 N $H_2SO_4$. The HPLC analysis shows concentration of LA in liquid phase is 24 g/l respectively.

Table 1. Reaction conditions for the LA production from technical lignin.

| Substrate2-G biomass residue/ Technical lignin | Catalyst | | Double distilled water | Reaction volume | Reaction conditions |
|---|---|---|---|---|---|
| | Conc. $H_2SO_4$ | $FeCl_3$ | | | |
| 200 g | 40 ml | 40 g | 720 ml | 1000 ml | 200 °C/1h |

**Example 2: Screening of solvents/diluents for purification of LA**

The acids containing liquid is mixed with different organic solvents/diluents at temperature (25 °C) with shaking (100 rpm) for 6 h as mentioned in Table 2. The LA is preferentially distributed in the organic solvent layer which is extracted using a separating funnel and the acidic aqueous liquid waste is processed separately. Among tested different solvents, including polar aprotic solvents, polar and non-polar solvents, fusel oil emerged as the best organic solvent for LA with the extraction yield of 76.63 %, as shown in Table 2. The composition of fusel oil mentioned in Table 3. The order of organic solvents which showed high compatibility for LA is: fusel oil >dichloromethane >diethyl ether > 1-butanol > 1-pentanol > ethyl acetate >hexane >chloroform >benzene >toluene. The organic phase is fractionally distilled to separate solvent and LA of high purity (98%). The extraction of the LA is also performed with individual components of fusel oil and with their synthetic blend. The slightly higher yield of the LA is achieved with fusel oil blend as shown in Table 4. The solvent is recycled for the next round of LA extraction and purification. The extraction efficiency is evaluated by the distribution constant (Kd) of LA in the organic phase [LA]org and in aqueous phase [LA] aq after sufficient mixing (Eq. 1). The extraction yield was defined by Equation 2.

$$Kd = \frac{[LA\ or\ FA]org}{[LA\ or\ FA]\ aq} \qquad\qquad Eq.\ 1$$

$$Extraction\ yield\ (E\%) = \frac{K}{1+K} x\ 100 \qquad\qquad Eq.\ 2$$

Table 2. Distribution constant (Kd) and extraction yield (E%) of LA at 25 °C for 10 different organic solvents.

| Solvents | LA | | | |
|---|---|---|---|---|
| | LA (org) | LA (aq) | Dissociation Constant (Kd) | Extraction Yield (%) |
| Diethylether | 14.5 | 9.5 | 1.52 | 60.31 |
| Dichloromethane | 16.4 | 7.6 | 2.1 | 67.74 |
| Chloroform | 4 | 20 | 0.2 | 16.66 |
| Ethylacetate | 11.5 | 12.5 | 0.92 | 47.91 |
| Hexane | 4.8 | 19.2 | 0.25 | 20.00 |

(continued)

| Solvents | LA | | | |
|---|---|---|---|---|
| | LA (org) | LA (aq) | Dissociation Constant (Kd) | Extraction Yield (%) |
| Toluene | 1.7 | 22.3 | 0.07 | 6.54 |
| Fusel oil | 18.4 | 5.6 | 3.28 | 76.63 |
| Benzene | 3.4 | 20.6 | 0.16 | 13.79 |
| 1-Butanol | 12.2 | 11.8 | 1.03 | 50.93 |
| 1-Pentanol | 11.8 | 12.2 | 0.96 | 48.97 |

Table 3. Chemical composition of fusel oil obtained in ethanol production unit.

| S. No. | Components | Content (%, v/v) |
|---|---|---|
| 1 | Iso-amyl alcohol | 55-60 |
| 2 | Iso-butyl alcohol | 18-20 |
| 3 | Active amyl alcohol | 10-15 |
| 4 | Butyl alcohol | 5 - 8 |
| 5 | Propyl alcohol | 5 - 8 |
| 6 | Hexanol | 2 - 5 |
| 7 | Others (including mixture of methanol, ethanol, acids, water, metal salts etc.) | 5 |

Table 4. Distribution constant (Kd) and extraction yield (E %) of LA at 25 °C with fusel oil components and its synthetic blend.

| Solvents | LA | | | |
|---|---|---|---|---|
| | LA (org) | LA (aq) | Dissociation Constant (Kd) | Extraction Yield (%) |
| Isoamylalcohol(IAA) | 10.4 | 13.6 | 0.76 | 43.44 |
| Isobutylalcohol(IBA) | 11.8 | 12.2 | 0.96 | 49.50 |
| Activeamylalcohol(AAA) | 6.0 | 18 | 0.33 | 25.06 |
| Butylalcohol(BA) | 11.7 | 12.3 | 0.95 | 48.78 |
| Propylalcohol(PA) | 6.4 | 17.6 | 0.36 | 26.73 |
| Hexanol(H) | 8.9 | 15.1 | 0.58 | 37.30 |
| Synthetic fuel oil blend IAA(55%)+IBA(18%)+AAA(10%)+ BA(5%)+PA (5%)+H(2%) | 18.9 | 5.1 | 3.70 | 78.72 |

**Example 3: Reactive extraction of LA using amines with various organic solvents/diluents**

The reactive extraction involves a reversible reaction between the acid in the aqueous phase and the extractant in the organic phase (diluent phase) which results in the formation of extractant-acid complex having high affinity for the organic phase. Two amines extractants namely octylamine and N-methyldioctylamine investigated in various combinations with 10 different organic solvents yielding 20 types of reactive extraction systems for the purification of LA (Table 5). The resulted dissociation constant (Kd) and extraction yield (E%) are summarized in Table 5 for various amine diluent systems.

**Table 5.** Distribution constant (Kd) and extraction yield (E%) of LA at 25 °C for 10 different organic solvents with amines, octylamine (0.2M) and N-methyldioctylamine (0.2M).

| Solvents | Octylamine | | | | N-methyldioctylamine | | | |
|---|---|---|---|---|---|---|---|---|
| | LA (org) | LA (aq) | Dissociation Constant (Kd) | Extraction yield (%) | LA (org) | LA (aq) | Dissociation Constant (Kd) | Extraction yield (%) |
| Diethylether | 14.8 | 9.2 | 1.6 | 61.53 | 16.2 | 7.8 | 2.07 | 67.42 |
| Dichloromethane | 17.3 | 6.7 | 2.58 | 72.06 | 20.3 | 3.7 | 5.48 | 84.56 |
| Chloroform | 4 | 20 | 0.2 | 16.66 | 4.7 | 19.3 | 0.24 | 19.35 |
| Ethylacetate | 12.2 | 11.8 | 1.03 | 50.73 | 13.3 | 10.7 | 1.24 | 55.35 |
| Hexane | 5.2 | 18.8 | 0.27 | 21.25 | 5.5 | 18.5 | 0.29 | 22.48 |
| Toluene | 1.8 | 22.2 | 0.081 | 7.5 | 1.8 | 22.2 | 0.081 | 7.4 |
| Fuseloil | 19.5 | 4.5 | 4.33 | 81.23 | 21.2 | 2.8 | 7.57 | 88.33 |
| Benzene | 3.6 | 20.4 | 0.17 | 14.52 | 3.9 | 20.1 | 0.194 | 16.24 |
| 1-Butanol | 14.3 | 9.7 | 1.47 | 59.51 | 15.1 | 9.9 | 1.52 | 60.31 |
| 1-Pentanol | 12.6 | 11.4 | 1.1 | 52.38 | 13.5 | 10.5 | 1.28 | 56.14 |

EP 4 458 798 A1

Evaluation of these system shows highest extraction yield (E%) (LA 88.33%) is obtained when fusel oil was used as a diluent with N-methyldioctylamine.

**Example 4: Influence of varying amine concentration on reactive extraction**

As mentioned above, not only the nature of amine and diluent but also the concentration of amine can influence the equilibrium extraction characteristics. The influence of varying amine concentration (0.2-1.0 M) on the reactive extraction of LA is investigated and the results are mentioned in Table 6. An increase in extraction yield (E %) of LA (95%) was observed with increase amine concentration up to 0.6 M. Further increase in amine concentration did not help to improve the extraction yield (E%).

Table 6. Effect of varying amine concentration on extraction yield of LA.

| N-methyldioctylamine concentration (M) | LA | | | |
|---|---|---|---|---|
| | LA (org) | LA (aq) | Dissociation Constant (Kd) | Extraction Yield (%) |
| 0.2 | 21.3 | 2.7 | 7.88 | 88.73 |
| 0.4 | 22.1 | 1.9 | 11.63 | 92.08 |
| 0.6 | 22.8 | 1.2 | 19.0 | 95.00 |
| 0.8 | 23.0 | 1.0 | 23.0 | 95.83 |
| 1.0 | 23.0 | 1.0 | 23.0 | 95.83 |

**Example 5: Catalyst recycling for improved titer and cost reduction**

The raffinate or acidic aqueous liquid waste generated after organic solvent extraction is recycled for the next batch of LA production. This strategy helped in reducing the catalyst costs by more than half and improved the titer of the LA. The experimental details and results are depicted in Table 7. Furthermore, spiking by adding the small amount of the catalyst (2.0%) during the LA production improved the titer.

Table 7. Raffinate recycling for enhanced titer and costs reduction

| Cycle | Substrate | Catalyst | LA titer (g/l) |
|---|---|---|---|
| 1 | Technical lignin | 4 % $H_2SO_4$ + 4 % $FeCl_3$ | 23.47 |
| 2 | Technical lignin | Raffinate generated from cycle 1 + 0.5% $H_2SO_4$ + 0.5 % $FeCl_3$ | 24.95 |
| 3 | Technical lignin | Raffinate generated from cycle 1 + 1% $H_2SO_4$ + 1 % $FeCl_3$ | 26.43 |
| 4 | Technical lignin | Raffinate generated from cycle 1 + 2% $H_2SO_4$ + 2 % $FeCl_3$ | 28.01 |
| 5 | Technical lignin | Raffinate generated from cycle 1 + 4% $H_2SO_4$ + 4 % $FeCl_3$ | 27.80 |

**Advantage of the method disclosed in the present invention for extraction and purification of levulinic acid over prior art:**

Prior art is applicable to purify the LA by utilizing costly chemicals viz. alkyl phenols, ketones, alcohols, fatty acids, esters, ether, halogenated hydrocarbons, ionic liquids, furfural or their combinations thereof, whereas the present invention disclosed a method to isolate high purity LA (98%), using cheap and waste solvent.

Fusel oil used for LA purification in this invention is generated as ethanol distillation waste streams during the 2G bioethanol production. This has potential to improve the cost-effectiveness of the process.

The N-methyldioctylamine used in this invention is highly efficient for recovery of LA in fusel oil or organic phase from aqueous solution.

This process is suitable to remove any colour and humins (soluble tar), which would otherwise create a problem in subsequent process step, particularly in distillation.

The process is easy to use and leads to higher extraction yields of LA (95.0 %) which is easily separated by fractional distillation.

The solvent could be easily recovered by fractional distillation and reused many times without any substantial loss in the efficiency.

The raffinate of the acid extraction is recycled back to the reaction step multiple times, which reduces the use of additional catalyst and make the overall process cost effective.

This invention shall improve the profitability of 2G biorefinery by utilizing the cheap waste streams generated indigenously in the distillation process for LA purification in a circular and integrated manner.

**Claims**

1. A novel integrated process for the extraction and purification of levulinic acid (LA) from a technical lignin, wherein the said process comprises of:

    i. heating the technical lignin with a catalyst in water into a reactor to obtain a supernatant containing LA and a black solid insoluble humins fraction;
    ii. separating the supernatant containing LA and the black solid insoluble humins fraction through centrifugation;
    iii. mixing of the supernatant containing LA with an organic solvent and an amine extractant;
    iv. purifying LA by a reactive extraction using the amine extractant with the organic solvent and separating an acidic aqueous liquid waste or raffinate to obtain a LA rich organic phase,
    wherein the reactive extraction involves a reversible reaction between the LA taken in the aqueous phase and the amine extractant in the organic phase which results in the formation of amine extractant-acid complex having high affinity for the organic phase;
    v. recycling the acidic aqueous liquid waste or raffinate generated after organic solvent extraction for the next batch of producing LA;
    vi. fractionally distilling the LA rich organic phase to separate the organic solvent and LA; and
    vii. recycling the organic solvent for the next round of extraction and purification.

2. The process as claimed in claim 1, wherein the technical lignin is bioethanol residue generated from a lignocellulosic biomass after 2G bioethanol processing.

3. The process as claimed in claim 2, wherein the lignocellulosic biomass is selected from agricultural residue, grass, fiber process residue, sugar cane straw, sorghum, forestry waste, hardwood, softwood, sawdust, crops or lignocellulosic material and a combination thereof; wherein the agricultural residue is selected form corn stover, wheat straw, cotton stalk, rice straw, canola straw, soybean stover and a combination thereof;

    the grass is selected form switchgrass, miscanthus, jatropha cuttings and a combination thereof; and
    the fiber process residue is selected form corn fiber, beet pulp, pulp mill fines and rejects, sugar cane bagasse and a combination thereof.

4. The process as claimed in any preceding claim, wherein the heating of the technical lignin in step (i) is performed at 50 - 300 °C for 60 - 180 minutes.

5. The process as claimed in any preceding claim, wherein the catalyst comprises a combination of 0.5 % - 4 % of concentrated $H_2SO_4$ and 0.5 % - 4 % of $FeCl_3$.

6. The process as claimed in any preceding claim, wherein the mixing of the supernatant containing LA with the organic solvent in step (iii) is done at a temperature of 25 - 30 °C for 30 - 240 minutes.

7. The process as claimed in any preceding claim, wherein the organic solvent is selected from fusel oil, dichloromethane, diethyl ether, 1-butanol, 1-pentanol, ethyl acetate, hexane, chloroform, benzene, toluene and a synthetic blend of fusel oil.

8. The process as claimed in any preceding claim, wherein components of fusel oil are iso amyl alcohol (IAA), iso butyl alcohol (IBA), active amyl alcohol (AAA), butyl alcohol (BA), propyl alcohol (PA), and hexanol (H) and a mixture of methanol, ethanol, acid, water, and metal salt.

9. The process as claimed in any preceding claim, wherein the synthetic blend of fusel oil comprises of iso amyl alcohol (55 - 60 %), iso butyl alcohol (18 - 20 %), active amyl alcohol (10 - 15 %), butyl alcohol (5-8 %), propyl alcohol (5-8

%), and hexanol (2-5 %).

10. The process as claimed in any preceding claim, wherein the amine extractant is selected from octylamine and N-methyldioctylamine.

11. The process as claimed in any preceding claim, wherein the concentration of the amine extractant is in the range of 0.2 - 1.0 M.

12. The process as claimed in any preceding claim, wherein the amine extractant-acid complex is selected from octylamine-LA complex and N-methyldioctylamine-LA complex.

13. The process as claimed in any preceding claim, wherein the technical lignin is obtained from pre-treatment of lignocellulosic biomass, comprising of:

i. treating lignocellulosic biomass with dilute acid selected from $H_3PO_4$, $HNO_3$, $CH_3COOH$, and $H_2SO_4$ having 0.02-3% w/w of the biomass for 5 - 60 min, at a temperature of 50 °C- 100 °C, followed by heating at 150 °C - 200 °C to obtain a slurry, to hydrolyse 40 % - 90 % of hemicellulose into sugars and to obtain minimum amounts of inhibitors selected from furfurals, hydroxy methyl furfural, levulinic acid, and acetic acids;
ii. cooling the slurry to the temperature of 35 - 50 °C to carry out the enzymatic hydrolysis and fermentation, either as separate hydrolysis and fermentation (SHF) or simultaneous saccharification and fermentation (SSCF) at a temperature in range of 35 °C - 50 °C to obtain a fermented broth; and
iii. distilling the fermented broth by standard distillation and molecular sieve methods to produce ethanol and technical lignin.

14. The process as claimed in claim 13, wherein the enzymatic hydrolysis and fermentation of lignocellulosic biomass is done with genetically modified yeast *saccharomyces cerevisiae.*

Europäisches
Patentamt

European
Patent Office

Office européen
des brevets

# EUROPEAN SEARCH REPORT

**Application Number**

EP 24 16 6036

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2015/063033 A1 (DSM IP ASSETS BV [NL]) 7 May 2015 (2015-05-07) * page 5, paragraph 2 - paragraph 3; claim 1 * * page 7, paragraph 1 * | 1-14 | INV. C07C51/00 C07C51/44 C07C51/48 C07C59/185 |
| Y | US 2006/047139 A1 (AYOUB PAUL M [NL]) 2 March 2006 (2006-03-02) * paragraph [0033] - paragraph [0036]; figures 1-2 * | 1-14 | |
| Y | US 2010/312006 A1 (LAKE MICHAEL A [US] ET AL) 9 December 2010 (2010-12-09) * paragraph [0045] - paragraph [0050]; figure 1 * | 1-14 | |
| Y | CN 107 915 619 A (CHINA PETROLEUM & CHEM CORP ET AL.) 17 April 2018 (2018-04-17) * paragraph [0027]; claim 1 * | 1-14 | |

**TECHNICAL FIELDS SEARCHED (IPC)**

C07C
C07B
C07G

The present search report has been drawn up for all claims

| Place of search | Date of completion of the search | Examiner |
|---|---|---|
| The Hague | 24 September 2024 | Seitner, Irmgard |

EPO FORM 1503 03.82 (P04C01)

## ANNEX TO THE EUROPEAN SEARCH REPORT
## ON EUROPEAN PATENT APPLICATION NO.

EP 24 16 6036

This annex lists the patent family members relating to the patent documents cited in the above-mentioned European search report.
The members are as contained in the European Patent Office EDP file on
The European Patent Office is in no way liable for these particulars which are merely given for the purpose of information.

24-09-2024

| Patent document cited in search report | | Publication date | Patent family member(s) | | Publication date |
|---|---|---|---|---|---|
| WO 2015063033 | A1 | 07-05-2015 | NONE | | |
| US 2006047139 | A1 | 02-03-2006 | AT | E396965 T1 | 15-06-2008 |
| | | | AU | 2005206337 A1 | 04-08-2005 |
| | | | BR | PI0507060 A | 19-06-2007 |
| | | | CA | 2554186 A1 | 04-08-2005 |
| | | | CN | 1914148 A | 14-02-2007 |
| | | | EP | 1723099 A1 | 22-11-2006 |
| | | | JP | 2007518778 A | 12-07-2007 |
| | | | PL | 1723099 T3 | 31-12-2008 |
| | | | US | 2006047139 A1 | 02-03-2006 |
| | | | WO | 2005070867 A1 | 04-08-2005 |
| US 2010312006 | A1 | 09-12-2010 | NONE | | |
| CN 107915619 | A | 17-04-2018 | NONE | | |

EPO FORM P0459

For more details about this annex : see Official Journal of the European Patent Office, No. 12/82

**EP 4 458 798 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014037560 A1
- WO 2014087015 A1
- WO 2017064069 A1
- US 2684982 A
- US 2257389 A